# FASCICULE DE BREVET EUROPEEN

(11) **EP 4 242 737 B1**
(45) Date de publication et mention de la délivrance du brevet: **10.12.2025**
(21) Numéro de dépôt: 23178277.2
(22) Date de dépôt: 17.10.2014
(51) Int. Cl.: G02C 13/00, A61B 3/00, G16H 10/60

(54) **PROCÉDÉ DE CONTRÔLE DE LA QUALITÉ DE MESURES D'OPTOMÉTRIE**
VERFAHREN ZUR QUALITÄTSKONTROLLE OPTOMETRISCHE MESSUNGEN
QUALITY CONTROL METHOD FOR OPTOMETRIC MEASUREMENTS

(30) Priorité: 08.11.2013 FR 1360989
(43) Date de publication de la demande: 13.09.2023
(62) Demande divisionnaire de: 14796827.5
(73) Titulaire: Essilor International, 94220 Charenton-Le-Pont (FR)
(72) Inventeur: HADDADI, Ahmed, 94220 Charenton-Le-Pont (FR); BERTHEZENE, Marie-Anne, 94220 Charenton-Le-Pont (FR); POULAIN, Isabelle, 94220 Charenton-Le-Pont (FR); PETIGNAUD, Cécile, 94220 Charenton-Le-Pont (FR); LEVRAUD, Loic, 94220 Charenton-Le-Pont (FR); GAYAT, Sébastien, 94220 Charenton-Le-Pont (FR); DIVO, Fabien, 94220 Charenton-Le-Pont (FR); ROUSSEAU, Benjamin, 94220 Charenton-Le-Pont (FR)
(74) Mandataire: Jacobacci Coralis Harle

(56) Documents cités:
- EP-A1- 2 466 540
- EP-A1- 2 600 157
- WO-A2-2013/087187
- US-A- 4 105 302
- US-A1- 2013 231 941

## Description

### DOMAINE TECHNIQUE AUQUEL SE RAPPORTE L'INVENTION

La présente invention concerne de manière générale le domaine de la fabrication de lentilles de compensation ophtalmique montées en lunettes de vue.

Elle concerne plus particulièrement un procédé de contrôle de la qualité de mesures d'optométrie utilisées pour déterminer les propriétés optiques de réfraction et la qualité de lentilles ophtalmiques adaptées à un porteur de lunettes.

### ARRIERE-PLAN TECHNOLOGIQUE

De manière classique, la fabrication de lentilles de compensation ophtalmique nécessite d'une part des mesures ophtalmiques pour déterminer en particulier les caractéristiques de réfraction oculaire des yeux d'une personne et d'autre part des mesures complémentaires, notamment de paramètres géométrico-morphologiques du porteur. Ces différentes mesures sont nécessaires pour la fabrication et le montage des lentilles ophtalmiques adéquates sur la monture choisie et pour l'ajustage des lunettes à la vue et à la morphologie du porteur.

Lors d'un examen oculo-visuel, un ophtalmologiste ou un optométriste effectue des mesures d'optométrie pour évaluer l'acuité visuelle d'une personne. Les mesures ophtalmiques peuvent être obtenues par différents appareils d'optométrie, en particulier un réfractomètre pour des mesures de réfraction oculaire. En cas de défaut d'acuité visuelle d'une personne, le résultat d'un examen oculo-visuel est souvent une prescription de lentilles ophtalmiques. Cette prescription indique généralement la correction de réfraction, souvent exprimée par des caractéristiques de puissance sphérique, cylindrique ou prismatique, d'axe, d'addition, de distance œil-lentille... nécessaire pour la fabrication de lentilles de compensation ophtalmiques adéquates pour chaque œil du porteur.

D'autre part, un optométriste ou un vendeur professionnel agréé tel qu'un opticien-conseil assure la distribution, le montage des lentilles ophtalmiques sur une monture de lunettes et l'ajustage des lunettes sur le porteur.

A cet effet, l'optométriste ou l'opticien effectue un ensemble de mesures complémentaires d'optométrie relatives à une personne. Le choix d'une monture détermine les dimensions physiques des lentilles. Les conditions d'utilisation des lunettes, telles que par exemple en vision de près, de loin et/ou intermédiaire, conduisent au choix de paramètres pour les lentilles ophtalmiques parmi une lentille monofocale, multifocale ou progressive. De plus, différentes options permettent de choisir le type et la qualité des lentilles ophtalmiques : matériau organique ou minéral, traitement de surface, par exemple anti-reflet, verre photochromique, gamme de prix...

L'opticien détermine aussi les propriétés géométrico-morphologiques du porteur, en particulier les distances inter-pupillaires ou encore la hauteur du ou des foyers par rapport au bord inférieur de la monture. Différents appareils d'optométrie peuvent servir aux mesures d'optométrie relatives aux propriétés géométrico-morphologiques du porteur, tels qu'un autoréfractomètre, un appareil de photoréfraction, une colonne ou une tablette de prise de mesure. De préférence, les appareils d'optométrie permettent des mesures dans une posture naturelle ergonomique pour le porteur et dans des conditions visuelles bien déterminées. Avantageusement, certains appareils permettent des mesures d'optométrie selon plusieurs postures habituelles du porteur correspondant par exemple à des conditions de vision naturelles telles que la vision de près, de loin et/ou intermédiaire.

Dans le présent document on entend par mesure d'optométrie, une mesure relative à un ou plusieurs paramètres ophtalmiques d'un porteur, en particulier de réfraction oculaire, et/ou à une ou plusieurs propriétés géométrico-morphologiques du porteur, en particulier les distances inter-pupillaires ou encore la hauteur des axes visuels par rapport au bord d'une monture, la mesure des angles pantoscopiques, de la distance verre-œil (DVO), de la position du centre de rotation de l'œil (CRO), du galbe de la monture, ou encore de paramètres comportementaux tels que le coefficient œil-tête, la distance de lecture, l'abaissement du regard en lecture.

Selon les lois en vigueur dans les différents pays, les mesures d'optométrie de type ophtalmique et de type géométrico-morphologique peuvent être effectuées par un même professionnel habilité ou au contraire doivent être effectuées par différents professionnels ayant chacun des activités réservées.

L'ensemble des mesures d'optométrie de type ophtalmique, géométrico-morphologique ou autre permet de déterminer la réfraction des lentilles et de calculer la courbure de base des lentilles ophtalmiques. La précision des mesures de ces paramètres géométrico-morphologiques du porteur est essentielle pour assurer le centrage des lentilles de compensation ophtalmique montées en lunettes par rapport aux axes visuels du porteur.

L'optométriste ou l'opticien valide ensuite le lancement de la gamme de fabrication des lentilles ophtalmiques pour lunettes auprès d'un fabricant. Après réception des lentilles ophtalmiques adéquates, l'opticien réalise le montage des lentilles ophtalmiques sur la monture de lunettes choisie par le porteur et l'ajustage des lunettes sur le visage du porteur.

Toutefois, on constate que les mesures d'optométrie sont de précision variable suivant les instruments utilisés, le soin apporté aux mesures et suivant les qualifications professionnelles respectives des optométristes, opticiens ou autres professionnels autorisés intervenant dans les différentes étapes. Or, les résultats de mesure ne sont généralement pas transmis avec une marge d'erreur.

De plus, certaines mesures d'optométrie ou de paramètres géométrico-morphologiques ne correspondent pas à une définition uniforme. Par exemple, l'angle pantoscopique d'une lentille représente l'angle que forme le plan général d'une lentille au porté par rapport à la verticale. Cependant, la convention de signe de l'angle pantoscopique peut varier suivant l'appareil ou le centre de mesure. Un même angle pantoscopique peut ainsi être évalué à +8 degrés ou -8 degrés. De telles différences de mesure sont aussi une source d'erreurs.

Des erreurs sur les paramètres de réfraction optique des lentilles ophtalmiques, sur les paramètres géométrico-morphologiques, sur le centrage ou sur l'ajustage de lentilles de compensation ophtalmique peuvent, dans certains cas, générer des complications oculaires pour le porteur, un inconfort visuel, des maux de tête ou des nausées et plus généralement entraîner une insatisfaction du porteur de lunettes.

D'autre part, il existe un besoin de traçabilité des mesures d'optométrie pour permettre d'adapter la qualité des lentilles ophtalmiques destinées à un porteur de lunettes.

Le document US2013/231941 décrit un système automatique de distribution de lunettes ophtalmiques qui permet à partir d'une image du patient de déterminer certaines mesures faciales. Le document EP 2 466 540 décrit un système de supervision et de maintenance à distance d'instruments médicaux.

### OBJET DE L'INVENTION

Afin de remédier à l'inconvénient précité de l'état de la technique, la présente invention propose un procédé de contrôle de la qualité de mesures d'optométrie pour la détermination des propriétés opto-mécaniques et de la qualité d'une lentille correctrice de lunettes adaptée à un porteur de lunettes selon la revendication 1.

D'autres caractéristiques non limitatives et avantageuses du procédé de contrôle de la qualité de mesures d'optométrie conforme à l'invention sont les suivantes :
- l'étape (d) comprend une étape de calibration dudit résultat de mesure d'optométrie ;
- l'étape (d) comprend l'étape suivante :
   (d1) affecter au moins un critère d'évaluation au résultat de mesure d'optométrie, le critère d'évaluation étant fonction des valeurs du premier enregistrement ; et/ou
   (d2) affecter au moins un même critère d'évaluation à une grappe (ou cluster de base données) associée à une pluralité d'appareil d'optométrie et/ou à une pluralité de sites de mesure d'optométrie
- le procédé comprend en outre une étape de transmission du au moins un critère d'évaluation au premier site de mesure d'optométrie et/ou à l'appareil d'optométrie ou respectivement à ladite pluralité d'appareil d'optométrie et/ou à ladite pluralité de sites de mesure d'optométrie ;
- l'étape (d1) ou (d2) comporte une étape d'enregistrement du critère d'évaluation dans une base de données ;
- le premier enregistrement informatique est enregistré dans une base de données à l'issue de l'étape (a), l'ensemble de données numériques de mesure est enregistré dans la même base de données à l'issue de l'étape (b) et le résultat du traitement numérique est enregistré dans la même base de données à l'issue de l'étape (d) ;
- l'étape (c) de transmission de l'ensemble de données numériques de mesure comporte une étape d'enregistrement d'une signature numérique dans le premier enregistrement de la base de données ;
- le procédé comprend en outre une étape de calcul d'un écart entre le résultat de mesure d'optométrie et le référentiel de données numériques et une étape de transmission de cet écart au premier site de mesure d'optométrie et/ou à l'appareil d'optométrie ;
- le référentiel de données numériques comprend au moins un critère d'évaluation représentatif du respect d'un protocole de mesure préétabli, de la reproductibilité de l'appareil d'optométrie, des qualifications d'un technicien pilotant l'appareil d'optométrie, du type d'appareil d'optométrie et/ou de la complexité des mesures d'optométrie ;
- le procédé comprend en outre au moins une autre exécution du procédé de la revendication 1, cette autre exécution étant associée à la même valeur de premier identifiant correspondant au même porteur de lunettes ;
- le procédé comprend une étape de certification du premier et/ou du deuxième site en fonction d'une signature numérique attribuée respectivement au premier et/ou au deuxième site.

De façon avantageuse, selon un mode de réalisation particulier, le procédé comprend en outre les étapes suivantes :
(e) déterminer un ensemble de données numériques de prescription de correction visuelle d'une nouvelle lentille correctrice (en particulier les propriétés opto-mécaniques et la qualité d'une lentille correctrice de lunettes) en fonction de l'ensemble de données numériques des mesures signées,
(f) faire valider et signer numériquement l'ensemble de données numériques de prescription de correction visuelle par un professionnel habilité à prescrire les puissances de réfraction de la nouvelle lentille correctrice,
(g) transmettre à un troisième site l'ensemble de données numériques de prescription, relié au premier identifiant du porteur et à une signature attachée au professionnel habilité,
(h) certifier la signature transmise à l'étape précédente et transmettre le résultat de cette certification au troisième site.

De préférence, le procédé comprend la ou les étape(s) suivante(s) :
(j) sélectionner le design optique (conception optique et/ou forme) ou la catégorie du design optique ou adapter le calcul du design optique en fonction de la signature attachée au professionnel habilité.
(k) enregistrer informatiquement un fichier image d'une prescription préexistante de correction visuelle du porteur associé au premier identifiant du porteur ;
(l) transmettre au second site un ensemble de données numériques de mesure comprenant le résultat de la mesure de l'étape (b) et le fichier image d'une prescription préexistante associé au premier identifiant du porteur et à une signature attachée au premier site ou à l'appareil d'optométrie ;
(m) déterminer par traitement du fichier image d'une prescription préexistante l'ensemble de données numériques de prescription de correction visuelle comprenant les trois puissances de réfraction (sphère, cylindre, axe) de la nouvelle lentille correctrice ;
(n) déterminer une gamme de fabrication et/ou une gamme de design d'une lentille correctrice de lunettes adaptée au porteur en fonction de la valeur du critère d'évaluation.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente schématiquement l'architecture d'un système pour l'acquisition de mesure d'optométrie, le traitement des mesures d'optométrie et la fabrication de lunettes de compensation ophtalmique mettant en œuvre le procédé de l'invention ;
- la figure 2 représente schématiquement un diagramme d'étapes d'un procédé selon un mode de réalisation de l'invention ;
- la figure 3 représente schématiquement un exemple de représentation graphique de plusieurs critères d'évaluation des mesures d'optométrie.

### Dispositif

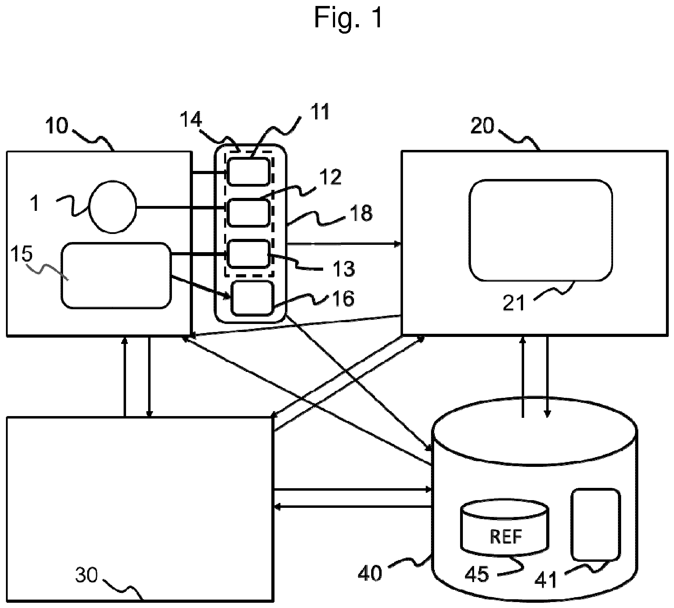

La figure 1 représente schématiquement l'architecture d'un système multi-site pour l'acquisition de mesures d'optométrie, le traitement de mesures d'optométrie, le stockage de mesures et l'évaluation de ces mesures, et pour la fabrication de lunettes de compensation ophtalmique.

Un premier site d'optométrie 10 est le site où est effectuée la prise de mesure d'optométrie. Un deuxième site 20 est un site de traitement des mesures d'optométrie. Un troisième site 30 est un site de fabrication des lunettes de compensation ophtalmique. Enfin, des moyens de stockage informatiques 40 sont reliés à ces trois sites. Le premier site 10 peut être un magasin d'optique faisant partie d'une chaîne de plusieurs magasins utilisant tous des appareils et procédés de mesure identiques, proposant des gammes identiques de montures et de verres, les différents sites étant par exemple reliés en réseau informatique.

Un appareil d'optométrie 15 est situé sur le premier site d'optométrie 10. Un porteur de lunettes 1 se trouve sur le site de mesure d'optométrie 10 à proximité de l'appareil d'optométrie 15.

Par exemple, le premier site d'optométrie 10 représente un cabinet de consultation d'ophtalmologie, d'optométrie ou un magasin d'opticien. De manière alternative, le premier site d'optométrie 10 peut correspondre à un ordinateur muni d'un capteur d'image. Le premier site d'optométrie 10 est généralement fixe mais peut aussi être mobile comme par exemple dans le cas d'un véhicule sanitaire équipé d'au moins un appareil d'optométrie.

Un appareil d'optométrie 15 est de manière générale tout appareil d'optométrie tel qu'un réfractomètre, un auto-réfractomètre, ou tout appareil fournissant une mesure de paramètre géométrico-morpholologique, tel qu'un appareil de mesure de distance inter-pupillaire, par exemple un pupillomètre, une colonne ou tablette de prise de mesure type Visioffice ou m'eye Fit, ou encore un simple réglet.

Le fonctionnement de l'appareil d'optométrie 15 est généralement piloté par un opérateur qui peut être une personne habilitée, un optométriste, un ophtalmologue ou un opticien.

Un identifiant unique 13 est attribué à l'appareil d'optométrie 15 du premier site d'optométrie 10 pour permettre d'identifier l'appareil d'optométrie 15 utilisé. Avantageusement, l'identifiant unique 13 contient des informations sur le type d'appareil d'optométrie, la marque du fabricant, la date de fabrication, la date de la dernière calibration, ainsi que sur le type de mesures fournies par cet appareil d'optométrie. De plus, l'identifiant unique 13 peut contenir des informations sur une certification qualité de l'appareil d'optométrie et/ou du site d'optométrie 1. Par exemple, un fabricant de lentilles ophtalmiques ou de lunettes fournit à un réseau d'opticiens un appareil d'optométrie spécifique 15 qui bénéficie de la certification de ce fabricant. Dans un autre exemple, un organisme de certification se charge de contrôler la conformité de l'appareil d'optométrie 15 à une norme de qualité, telle que par exemple une certification par exemple suivant la norme ISO: 10342 sur les autoréfractomètres. Ainsi, une attestation de certification et une date de certification peuvent être enregistrées dans l'identifiant unique 13 de l'appareil d'optométrie 15. De manière optionnelle, l'identifiant unique 13 de l'appareil d'optométrie 15 est complété par un autre identifiant permettant de reconnaître l'opérateur qui effectue la prise de mesure sur cet appareil d'optométrie 15.

Avantageusement, un autre identifiant unique 11 est attribué au site d'optométrie 10 où se situe l'appareil d'optométrie 15. L'identifiant 11 peut aussi permettre d'identifier une chaîne de magasins d'optique qui utilisent tous les mêmes méthodes de mesure. De même, un autre identifiant unique 12 est attribué au porteur 1.

De façon préférée, on regroupe dans un identifiant global 14 les différents identifiants disponibles : l'identifiant 13 de l'appareil d'optométrie 15, l'identifiant 12 du porteur 1, l'identifiant 11 du site de mesure 10 et éventuellement l'identifiant de l'opérateur qui pilote la mesure d'optométrie.

Le premier site d'optométrie 10 peut accueillir plusieurs appareils d'optométrie 15, qui sont soit des appareils d'optométrie identiques fournissant des mesures analogues soit des appareils d'optométrie fournissant des mesures d'optométrie complémentaires. Par exemple, le site 10 comprend un appareil de mesure de front d'onde, un réfractomètre, un appareil de mesure de paramètres géométrico-morphologiques du porteur tels que la distance interpupillaire, la largeur du pontet nasal, la distance entre la face intérieure d'une lentille et la cornée de l'œil du porteur, la hauteur des pupilles par rapport au bord inférieur de lunettes, suivant une ou plusieurs directions du regard du porteur.

L'appareil d'optométrie génère une ou plusieurs mesure(s) d'optométrie 16 du porteur de lunettes. Le premier site 10 fournit ainsi un ensemble de données numériques 18 comprenant d'une part l'enregistrement informatique 14 du ou des identifiant(s) 11, 12, 13 respectifs du premier site 10, du porteur de lunettes 1, et de l'appareil d'optométrie, et d'autre part une ou plusieurs mesure(s) d'optométrie 16. Le dispositif de l'invention permet ainsi de générer plusieurs enregistrements numériques, où chaque enregistrement numérique comporte au moins un identifiant 12 du porteur et une mesure d'optométrie 16 pour un couple : porteur de lunettes 1 et appareil d'optométrie 15.

Une base de données de référence 45 est de préférence enregistrée sur les moyens de stockage informatiques 40. La base de données de référence 45 comprend un ensemble de mesures effectuées sur un appareil d'optométrie 15 qui est identifié par son identifiant 13. La base de données de référence 45 peut comprendre un ensemble de mesures d'optométrie effectuées par un opérateur identifié. La base de données de référence 45 comprend un ensemble de mesures d'optométrie 16 associées à un porteur 1, chacune de ces mesures 16 étant assortie d'une date de mesure et attribuée à un appareil d'optométrie 15 identifié par son identifiant 13. La base de données de référence 45 peut aussi comprendre des statistiques de mesure d'optométrie pour différentes catégories de population en fonction de l'âge ou de diverses pathologies oculaires.

Chaque enregistrement numérique de la base de données se rapporte à un couple formé d'un porteur de lunettes 1 et d'un appareil d'optométrie 15 identifiés par leurs identifiants respectifs. Dans un exemple de réalisation chaque enregistrement numérique contient une mesure d'optométrie 16, une valeur d'identifiant du porteur et une valeur d'identifiant de l'appareil d'optométrie 15. Dans un autre exemple de réalisation chaque enregistrement numérique contient une mesure d'optométrie 16 et un lien informatique ou hyperlien relié à un autre registre contenant une valeur d'identifiant du porteur et une valeur d'identifiant de l'appareil d'optométrie 15. Dans encore un autre exemple de réalisation chaque enregistrement numérique contient un premier lien informatique vers un premier registre contenant une mesure d'optométrie 16 et un autre lien informatique vers à un autre registre contenant une valeur d'identifiant du porteur et une valeur d'identifiant de l'appareil d'optométrie 15. L'homme du métier est apte à implémenter d'autres modes de réalisation adéquats.

Des moyens informatiques permettent d'extraire de chaque enregistrement numérique la mesure d'optométrie enregistrée et la ou les valeurs d'identifiant(s) qui lui sont reliées informatiquement.

Un système de traitement informatique 21 situé sur un deuxième site 20 permet de traiter numériquement des mesures d'optométrie 16 issues de l'appareil d'optométrie 15. Le système de traitement 21 est relié à la base de données de référence 31. Le système de traitement 21 effectue un traitement des mesures d'optométrie en fonction des données enregistrées dans la base de données de référence 31.

Le système de traitement 21 peut être constitué d'un ordinateur et d'un système de traitement numérique des données, basé sur des algorithmes de traitement connus par ailleurs.

Un troisième site 30 correspond à une unité de fabrication de lunettes équipées de lentilles correctrices adaptées à la vue du porteur et à ses mesures géométrico-morphologiques.

De façon avantageuse, chacun des sites de mesure 10, de traitement des données 20, de fabrication 30 et de stockage informatique 40 est relié aux autres sites par des moyens de télécommunication. Les différents sites sont par exemple reliés entre eux par un réseau informatique. Un système informatique, par exemple basé sur un échange de jetons, permet d'assurer la sécurité des communications de flux de données entre les différents sites.

### Procédé

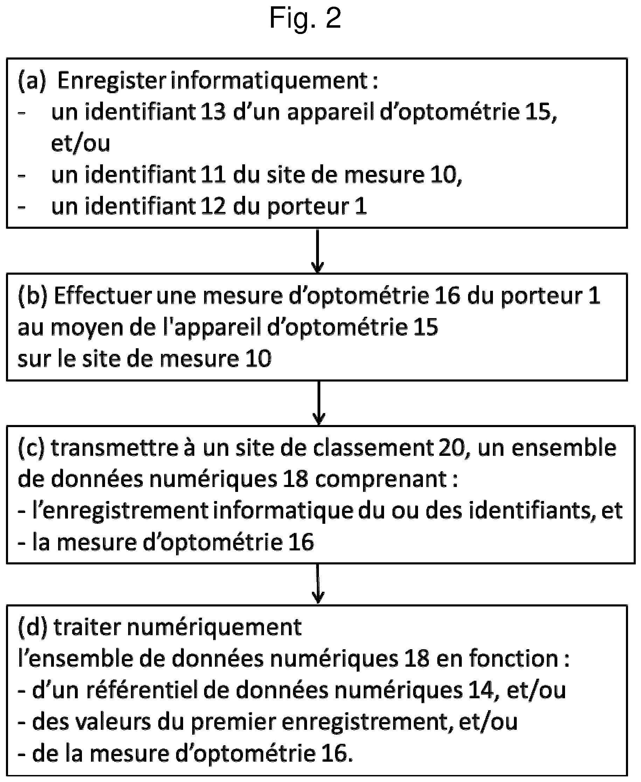

La figure 2 représente schématiquement un exemple d'étapes de procédé de contrôle de la qualité de mesures d'optométrie.

Dans une première étape a), on effectue un enregistrement informatique 14 comprenant au moins l'identifiant unique 13 attribué à l'appareil d'optométrie 15 du premier site d'optométrie 10. De manière complémentaire, l'enregistrement informatique 14 contient aussi l'identifiant 11 du site de mesure 10 et/ou respectivement l'identifiant 12 du porteur 1.

L'enregistrement informatique 14 du ou des identifiant(s) 11, 12, 13 est transmis vers une base de données 41 des moyens informatiques 40. De préférence, l'enregistrement informatique 14 comporte l'attribut d'une signature numérique permettant d'assurer la sécurité des échanges et de l'enregistrement dans la base de données 41.

Dans une étape b), on effectue au moins une mesure d'optométrie 16 sur un porteur de lunettes 1 au moyen d'un appareil d'optométrie 15 du premier site de mesure 10. La mesure d'optométrie 16 comprend par exemple une mesure de réfraction oculaire objective comprenant des valeurs de puissance sphérique, puissance cylindrique et d'axe relatives aux deux yeux du porteur. De manière alternative ou complémentaire, la mesure d'optométrie 16 comprend une mesure de la position verticale et horizontale de la pupille d'un œil du porteur par rapport à une monture destinée à accueillir une lentille correctrice.

La mesure d'optométrie 16 peut comprendre une mesure pour une posture déterminée ou une pluralité de mesures 161, 162 correspondant à plusieurs postures déterminées. Par exemple, une première mesure d'optométrie 161 en vision de loin correspond à une posture du porteur où le point de fixation du regard a une proximité inférieure à environ 2 dioptries et où l'axe du regard est horizontal. Une autre mesure d'optométrie 162 en vision de près correspond à une posture du porteur où le point de fixation du regard a une proximité comprise environ entre 2 et 5 dioptries et où l'axe du regard est incliné vers le bas de 30 à 60 degrés par rapport à une ligne horizontale.

Des informations complémentaires utiles pour la fabrication des lentilles montées en lunettes sont de préférence enregistrées et transmises conjointement avec la mesure d'optométrie 16. Ainsi, le choix par le client et/ou l'opérateur du type et de la qualité des lentilles ophtalmiques : matériau organique ou minéral, le choix d'un traitement de surface, par exemple anti-reflet, le choix d'un verre teinté ou photochromique, le choix d'une gamme de prix sont de préférence enregistrés pour être transmis non seulement au site de fabrication 30 mais aussi au site de traitement des données 20.

A l'issue de l'étape (b), un ensemble de données numériques 18 comprenant d'une part l'enregistrement informatique 14 du ou des identifiant(s) 11, 12, 13 et d'autre part une ou plusieurs mesure(s) d'optométrie 16, 161, 162 peut être transmis aux moyens de stockage 30 pour être enregistré dans la base de données 41.

L'ensemble de données numériques 18 peut comprendre des données de prescription de correction visuelle qui ne découlent pas nécessairement des mesures d'optométrie effectuées sur le site de mesure 10. Par exemple, les données de prescription peuvent être enregistrées à partir d'une prescription établie au préalable.

De façon avantageuse, l'ensemble de données numériques 18 et de prescription de correction visuelle sont validées et signées numériquement par un professionnel habilité à prescrire les puissances de correction de la ou des nouvelles lentilles de compensation.

Lors de l'étape (c), cet ensemble de données numériques 18 comprenant un enregistrement informatique 14 du ou des identifiant(s) 11, 12, 13 associé à une ou plusieurs mesure(s) d'optométrie 16, 161, 162 d'un porteur 1 est transmis au deuxième site 20 de traitement des mesures d'optométrie. De préférence, cette étape de transmission comporte une étape d'enregistrement d'une signature numérique dans la base de données. C'est notamment le cas lorsque seules des personnes disposant d'une signature numérique sont habilitées à transmettre des données numériques 18 vers d'un site de mesure 10 vers un site de traitement 20, un site de fabrication 30 ou vers des moyens de stockage 40.

Dans une étape (d), on effectue un traitement des données numériques 18 reçues par le site de traitement 20.

Plus précisément, le site de traitement 20 reçoit l'ensemble de données numériques 18 directement du site de mesure 10 ou extrait l'ensemble de données numériques 18 des moyens de stockage informatiques 41.

Ainsi, le système de traitement 21 accède à l'ensemble de données numériques 18 comprenant au moins une mesure d'optométrie 16 associée à l'enregistrement informatique 14 du ou des identifiant(s), notamment l'identifiant 13 de l'appareil d'optométrie 15 utilisé pour la mesure, l'identifiant 11 du site de mesure 10 et/ou respectivement l'identifiant 12 du porteur 1.

D'autre part, le système de traitement 21 accède à une base de données de référence 45.

En fonction des valeurs de l'enregistrement informatique 14 et de la base de données de référence 45, le système de traitement des données 21 effectue un traitement de la mesure d'optométrie 16. Ce traitement peut être constitué d'une ou de plusieurs opérations.

A titre d'exemple, un traitement des données comprend une étape de comparaison de la mesure d'optométrie avec un référentiel de mesures d'optométrie du même type enregistrées dans la base de données de référence 45. Le traitement comporte par exemple une comparaison de la mesure 16 avec une valeur de référence correspondant à un seuil, un minimum, un maximum ou une valeur moyenne. Connaissant l'identifiant 13 de l'appareil d'optométrie 16 et l'identifiant 12 du porteur 1 et, éventuellement, l'identifiant 11 du site de mesure 10, la valeur de référence utilisée pour la comparaison est une valeur spécifique liée à cet appareil de mesure 15 et à ce porteur 1 et, éventuellement, à ce site de mesure 10. Il est ainsi possible d'obtenir une traçabilité dans le temps des mesures d'optométrie relatives à un appareil de mesure 15 et à un porteur 1 et, éventuellement, à un site de mesure 10.

Le système de traitement des données peut aussi réaliser une mise en forme des données ou calibration des données afin de les remettre dans un référentiel standard. Par exemple, la mesure des angles pantoscopiques peut différer d'un appareil à un autre, ou d'un opérateur à un autre. Le système de traitement modifie ainsi les valeurs d'angles pantoscopiques afin qu'elles soient exprimées, indépendamment de l'appareil de mesure, dans un même référentiel de mesure. Les mises en forme ou calibration réalisées peuvent utiliser des décalages ou offset, des coefficients de proportionnalité, ou plus généralement toute loi de calibration permettant d'obtenir un passage d'un référentiel de mesure vers le référentiel standardisé. Cette calibration peut s'appliquer à toutes les mesures pour lesquels l'appareil ou l'opérateur ne fournit pas de valeurs standard (par exemple d'angle pantoscopique, de distance verre-œil (Dvo) ou de position du centre de rotation de l'œil (Cro), ou du galbe de la monture...). Les valeurs ainsi mises en forme ou calibrées peuvent être transmises vers le site de mesure 10 afin que celui-ci puisse réutiliser ces valeurs normalisées, par exemple pour les transmettre au site de fabrication 30.

Le traitement comprend une étape d'évaluation d'un critère de classement, basé sur la valeur de l'identifiant 13 de l'appareil de mesure 15, de l'identifiant du porteur 1 et, éventuellement, de l'identifiant 11 du site de mesure 10 ou encore sur un identifiant attribué à l'opérateur ayant effectué la mesure.

Par exemple, un appareil d'optométrie 15 est certifié pour délivrer des mesures avec une reproductibilité ou une marge d'incertitude déterminée. Un critère de classement ou critère de qualité ou critère d'évaluation peut ainsi être attribué en fonction de la reproductibilité de l'appareil d'optométrie. Par exemple, une série de mesures 16 de réfraction oculaire d'un porteur est comparée à la gamme de valeurs et à la reproductibilité d'un appareil d'optométrie : si cette série de mesures 16 est compatible avec la gamme de valeurs et de reproductibilité de référence, un critère d'évaluation peut être attribué à la mesure 16 de réfraction oculaire. Ce critère d'évaluation peut être transmis par le système de traitement au site de mesure. Dans le cas où la série de mesure est validée, on peut ensuite utiliser une valeur moyenne de la série de mesures 16 de réfraction oculaire. A contrario, si une série de mesures 16, effectuées successivement dans des conditions de reproductibilité, présente un écart supérieur à la reproductibilité de l'appareil de mesure 15 utilisé, une valeur de non validation de la mesure est générée par le système de traitement. De façon avantageuse, cette non validation est transmise au site de mesure 10. L'attribution d'un critère de « non validation » permet de déclencher une nouvelle prise de mesure ou un contrôle de l'appareil de mesure.

Dans un autre exemple, une mesure 16, par exemple de distance inter-pupillaire (IPD), est effectuée sur un appareil d'optométrie 15. Le système de traitement 21 compare cette mesure d'IPD à des valeurs statistiques de référence ou à une précédente mesure d'IPD du même porteur 1. Le résultat de cette comparaison est la détermination d'un écart. La valeur de cet écart peut être transmise au site de mesure 10. Selon que cet écart est inférieur ou supérieur à un seuil prédéterminé, le système de traitement 21 transmet au site de mesure 10 un critère de validation ou de non-validation de la mesure 16, qui peut ainsi être réitérée.

Dans certains cas, l'appareil ou l'opérateur peut ne pas être certifié pour délivrer des mesures avec une certaines reproductibilité ou marge d'incertitude, par exemple s'il s'agit d'un appareil inconnu ou nouveau.

La fourniture des mesures de l'appareil peut alors être comparée avec un seuil d'acceptation minimum, et ainsi on peut vérifier que la performance de mesure est suffisante pour accepter les mesures, et renvoyer au site 10 une information sur l'acceptation de ces mesures.

En fonction de la présence ou non d'un certificat de qualité attribué à un appareil d'optométrie, le système de traitement attribue un critère de qualité à la mesure d'optométrie.

Dans le présent document on entend par associer ou attribuer, l'action de créer un lien informatique entre des données informatiques. Ce lien informatique peut être un enregistrement commun dans une même base de données, ou un lien informatique entre des enregistrements distincts dans une ou plusieurs bases de données. Une valeur associée ou attribuée à un enregistrement est donc reliée informatiquement de manière univoque à cet enregistrement.

Un autre critère d'évaluation peut être basé sur le respect d'un protocole de mesure préétabli. Par exemple, une charte de qualité s'appliquant à un appareil de mesure 16 définit les étapes d'un protocole de mesure, les conditions de mesure, les réglages et/ou l'ordre d'exécution des différentes étapes (mesure en vision de loin, de près...) qui sont nécessaires pour obtenir des mesures conformes à une norme. L'enregistrement des mesures permet de vérifier l'ordre dans lequel les étapes de mesures ont été effectuées et pour chaque étape si les conditions d'exécution définies ont été respectées ou non. Un critère d'évaluation d'un protocole de mesure préétabli peut ainsi être affecté *a posteriori à* une mesure 16 ou à un ensemble de mesures d'optométrie. Le traitement peut permettre de contrôler la conformité des mesures effectuées avec un protocole de mesure prédéfini.

Dans encore un autre exemple, le critère d'évaluation attribué à la mesure 16 est basé sur les qualifications professionnelles de l'opérateur ayant effectué la mesure. Par exemple, si la mesure est effectuée par un personnel hautement qualifié, un critère de confiance élevé peut être attribué à cette mesure 16. A contrario, dans le cas où le porteur effectue lui-même une mesure d'IPD par l'intermédiaire d'une caméra d'ordinateur, un critère de confiance faible est attribué à cette mesure. Un retour au site 10 de ce critère peut être fait, ainsi que la cause expliquant la faible valeur du critère (problème de répétabilité, précision insuffisante, dynamique insuffisante...). Par rétroaction, le site 10 peut éventuellement déterminer des moyens correctifs afin d'atteindre un critère plus élevé.

Un critère d'évaluation peut aussi être lié au degré de complexité des mesures pour fournir une proposition de lunettes adaptée.

Un critère supplémentaire correspond par exemple à un taux de retour du fait d'un défaut de montage, de centrage ou de non adaptation des lunettes à la vue du porteur. Ce critère supplémentaire peut être affecté d'un facteur de pondération pour être combiné à une fonction de proposition de lunette adaptée ou de décision de type de lunette adaptée.

On peut créer une grappe (cluster) dans la base de données correspondant à un groupe d'équipements d'optométrie et/ou un groupe de mesures, ces groupes étant associés par exemple à un groupement de magasins d'opticiens. Ainsi, il est possible d'affecter à cette grappe (correspondant) un même critère de taux de retour, ou de taux d'équipement défaillant et/ou de protocole de mesure défaillant. Ce critère de taux de retour ou de défaillance peut être affecté simultanément à une grappe correspondant à une pluralité d'équipements et/ou de procédés de mesure défaillants ou non conformes.

Enfin, un autre critère d'évaluation peut être lié à l'identifiant 11 d'un site de mesure 10 déterminé, par exemple en fonction de la qualité des appareils d'optométrie disponibles ou de l'historique des mesures effectuées sur ce site de mesure 10. L'identifiant 11 du site de mesure 10 peut permettre d'identifier les adhérents d'un réseau professionnel de distribution de lunettes de compensation ophtalmique. Ce réseau est équipé d'une catégorie d'appareils d'optométrie particulière, qui est ainsi automatiquement reconnue. Prenons le cas d'un réseau de professionnels qui distribue en exclusivité pour un fabricant une certaine qualité de lentilles ophtalmiques, par exemple des lentilles multifocales ou progressives. En fonction de l'identifiant 11 du site de mesure, différents catalogues de verres sont alors rendus accessibles à la fabrication et la distribution par le site de mesure 10 identifié.

Le critère d'évaluation peut être un critère binaire. Ainsi un critère d'évaluation binaire correspond à la disponibilité ou non d'une mesure d'optométrie, telle qu'une mesure d'IPD, ou encore une mesure de réfraction différenciée en vision de près et en vision de loin. Un critère d'évaluation binaire peut aussi correspondre à la validation ou non-validation d'une mesure d'optométrie. Un autre type de critère d'évaluation peut prendre plusieurs valeurs sur une échelle.

La valeur du critère d'évaluation peut ensuite être transmise au système de stockage 40 pour enrichir une base de données statistique et/ou au site de mesure 10. Ainsi, le critère d'évaluation peut permettre de valider ou non une mesure de réfraction oculaire ou une mesure d'un paramètre géométrico-morphologique.

Le traitement d'une mesure 16 peut aussi être relié à plusieurs critères d'évaluation tels que les critères détaillés ci-dessus.

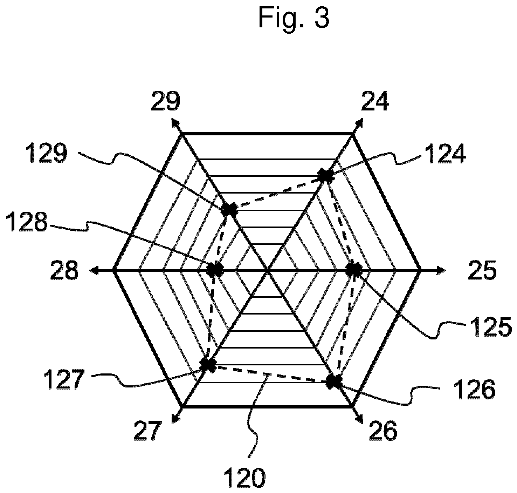

Ces différents critères d'évaluation sont par exemple représentés sur un diagramme en forme de radar, tel que sur la figure 3, où chaque rayon du radar représente l'échelle graduée d'un critère d'évaluation. Le nombre de rayons correspond au nombre de critères d'évaluation.

Chaque critère d'évaluation peut être lié à une mesure spécifique : par exemple un premier critère 24 est lié à la mesure de réfraction oculaire de l'œil droit du porteur 1, un deuxième critère 25 est lié à la mesure de réfraction oculaire de l'œil gauche, un troisième critère 26 est lié au respect d'un protocole de mesure appliqué à l'appareil de réfractométrie, un quatrième critère 27 est lié à une mesure d'écart inter-pupillaire du porteur, un cinquième critère 28 est lié à la qualité d'une mesure de hauteur d'axe visuel droit par rapport au bord inférieur d'une monture, et un sixième critère 29 est lié à la qualité d'une mesure de hauteur d'axe visuel gauche par rapport au bord inférieur d'une monture.

Alternativement, une seule et même mesure peut être liée à plusieurs critères d'évaluation tels que : la reproductibilité de cette mesure, le respect du protocole de mesure, l'écart relatif à une mesure antérieure du même porteur...

La valeur de chaque critère d'évaluation est représentée par un repère sur le rayon correspondant. Un tel graphique permet de visualiser une valeur moyenne ou des valeurs de seuil, minimum ou maximum, pour chacun des critères d'évaluation.

Dans le cas où plusieurs critères d'évaluation sont calculés, un coefficient de pondération est avantageusement affecté à chaque critère d'évaluation.

Une évaluation multi-critères permet de comparer globalement l'évaluation des mesures avec des références de mesures types préétablies. On peut par exemple calculer une aire sur un graphique en toile d'araignée, l'aire étant fonction d'une qualité globale des mesures.

Les résultats de l'évaluation mono ou multi-critères sont transmis au site de fabrication 30. Ces résultats sont aussi transmis aux moyens de stockage 30, dans un but de traçabilité des mesures ou pour compléter une base de données statistiques.

En fonction à la fois des mesures d'optométrie et du résultat de l'évaluation de ces mesures d'optométrie, le fabricant peut déterminer une gamme de fabrication et/ou une gamme de design la mieux adaptée aux besoins du porteur. De préférence, des domaines définis par des valeurs limites sont enregistrées pour un ou plusieurs critères d'évaluation. Ces domaines sont associés informatiquement à différentes gammes de fabrication et/ou gammes de design de lentilles correctrices de lunettes d'un fabricant.

Par exemple, pour une prescription de correction oculaire donnée reliée à une évaluation des mesures de qualité moyenne, le fabricant propose des verres dans une gamme de qualité moyenne et/ou dans une catégorie de design prédéfinie compatible avec la prescription requise.

Par exemple, on peut choisir un design progressif personnalisé ou un design progressif générique, ou un design progressif lié à l'activité du porteur, ou un type de verre particulier (SV/DBF/PAL).

Dans le cas d'une prescription de correction ophtalmique reliée à une évaluation des mesures d'excellente qualité, le fabricant peut proposer des verres personnalisés ayant des caractéristiques extrêmement précises et/ou dans une catégorie de design haut de gamme. Dans ce cas, le fabriquant peut proposer des verres progressifs dont les positions des foyers sont déterminées de manière très précise.

Inversement, pour une prescription de correction oculaire donnée qui est assortie à des mesures d'optométrie évaluées avec un degré de qualité faible, il est préférable d'orienter la fabrication vers des verres de correction présentant des tolérances de paramétrage élevées, qui sont compatibles avec les marges d'erreur liées à la faible qualité des mesures d'optométrie.

Le site de fabrication 30 définit ainsi une proposition de lunettes équipés de verres compatibles avec les mesures d'optométrie peut ensuite être transmise du site de fabrication 30 au site de mesure 10. L'opticien ou l'opérateur du site de mesure 10 peut ainsi proposer au porteur une ou plusieurs paires de lunettes compatibles avec les mesures effectuées et avec les contraintes de fabrication.

Après validation du choix de lunettes par le porteur, la fabrication des lunettes peut être déclenchée.

La gamme de fabrication des verres est de préférence enregistrée sur les moyens de stockage informatiques 30.

Ce procédé permet ainsi d'adapter la fabrication des lunettes non seulement en fonction de la correction requise, mais aussi en fonction d'une évaluation de la qualité des mesures d'optométrie effectuées pour ajuster les verres de compensation à la monture de lunettes choisie.

## Revendications

1. Procédé de contrôle de la qualité de mesures d'optométrie pour la détermination des propriétés opto-mécaniques et de la qualité d'une lentille correctrice de lunettes adaptée à un porteur de lunettes, le procédé de contrôle comprenant les étapes suivantes :
(a) enregistrer informatiquement un premier enregistrement (14) comprenant au moins une première valeur (12) d'un premier identifiant permettant l'identification d'un porteur de lunettes (1) et au moins une autre valeur (13) d'un autre identifiant permettant l'identification d'un appareil d'optométrie (15) sur un premier site de mesure d'optométrie (10) ;
(b) effectuer, au moyen de l'appareil d'optométrie (15) du premier site (10), au moins une mesure d'optométrie (16) comprenant au moins une mesure d'un paramètre de réfraction oculaire du porteur de lunettes (1) et/ou une mesure des positions verticale et horizontale de la pupille de l'œil de ce porteur par rapport à une monture destinée à accueillir la lentille correctrice, ;
(c) transmettre à un deuxième site (20) un ensemble de données numériques de mesure (18) comprenant le résultat de mesure d'optométrie (16) de l'étape (b), l'ensemble de données numériques de mesure (18) étant relié informatiquement au premier enregistrement (14) ;
(d) traiter numériquement le résultat de mesure d'optométrie (16) relié informatiquement au premier enregistrement (14) en fonction d'un référentiel de données numériques (45) et des identifiants respectifs du porteur de lunettes (12) et de l'appareil d'optométrie (13) du premier enregistrement (14), le référentiel de données numériques comprenant un référentiel de mesures d'optométrie du porteur de lunettes (12) et de l'appareil d'optométrie (15), et le traitement numérique comprenant une comparaison et détermination d'un écart entre ladite mesure d'optométrie (16) et le référentiel de mesures d'optométrie, pour affecter soit un critère de validation soit un critère de non-validation à ladite mesure d'optométrie (16) selon que cet écart est inférieur ou supérieur à un seuil prédéterminé ;
(g) transmettre au premier site (10) ledit critère de non-validation pour réitérer ladite mesure d'optométrie (16) ou, respectivement, le critère de validation pour accepter ladite mesure d'optométrie (16);
(i) générer sur le troisième site (30) un ensemble de données numériques de commande de lentille correctrice déclenchant la fabrication de ce verre.

2. Procédé selon la revendication 1 dans lequel l'étape (d) comprend une étape de calibration dudit résultat de mesure d'optométrie (16).

3. Procédé selon l'une des revendications 1 à 2 dans lequel l'étape (d) comporte une étape d'enregistrement du critère d'évaluation dans une base de données (41).

4. Procédé selon l'une des revendications 1 à 3 dans lequel le premier enregistrement (14) est enregistré dans une base de données (41) à l'issue de l'étape (a), l'ensemble de données numériques de mesure (18) est enregistré dans la même base de données (41) à l'issue de l'étape (b) et le résultat du traitement numérique est enregistré dans la même base de données (41) à l'issue de l'étape (d).

5. Procédé selon l'une des revendications 3 ou 4 dans lequel l'étape (c) de transmission de l'ensemble de données numériques de mesure (18) comporte une étape d'enregistrement d'une signature numérique dans le premier enregistrement (14) de la base de données.

6. Procédé selon l'une des revendications 1 à 5 comprenant en outre au moins une autre exécution du procédé de la revendication 1, cette autre exécution étant reliée à la même valeur de premier identifiant correspondant au même porteur de lunettes.

7. Procédé selon l'une des revendications 1 à 6 comprenant en outre les étapes suivantes :
(e) déterminer un ensemble de données numériques de prescription de correction visuelle d'une nouvelle lentille correctrice en fonction de l'ensemble de données numériques des mesures signées,
(f) faire valider et signer numériquement l'ensemble de données numériques de prescription de correction visuelle par un professionnel habilité à prescrire les puissances de réfraction d'une nouvelle lentille correctrice,
(g) transmettre à un troisième site (30) l'ensemble de données numériques de prescription, associé au premier identifiant (12) du porteur et à une signature attachée au professionnel habilité,
(h) certifier la signature transmise à l'étape g) et transmettre le résultat de cette certification au troisième site.

8. Procédé selon l'une des revendications 1 à 7 comprenant en outre l'étape suivante :
(j) sélectionner le design optique ou la catégorie du design optique ou adapter le calcul du design en fonction de la signature attachée au professionnel habilité.

9. Procédé selon la revendication 8 comprenant en outre les étapes suivantes :
(k) enregistrer informatiquement un fichier image d'une prescription préexistante de correction visuelle du porteur associé au premier identifiant du porteur ;
(l) transmettre au second site un ensemble de données numériques de mesure comprenant le résultat de la mesure de l'étape (b) et le fichier image d'une prescription préexistante associés au premier identifiant du porteur et à une signature attachée au premier site ou à l'appareil d'optométrie ;
(m) déterminer par traitement du fichier image d'une prescription préexistante l'ensemble de données numériques de prescription de correction visuelle comprenant les trois puissances de réfraction de la nouvelle lentille correctrice.

## Patentansprüche

1. Verfahren zur Kontrolle der Qualität optometrischer Messungen zur Bestimmung der optomechanischen Eigenschaften und der Qualität einer Brillenkorrekturlinse, die an einen Brillenträger angepasst ist, wobei das Kontrollverfahren die Folgenden Schritte beinhaltet:
(a) datentechnisches Registrieren einer ersten Registrierung (14), die mindestens einen ersten Wert (12) einer ersten Kennung, der die Identifikation eines Brillenträgers (1) gestattet, und mindestens einen anderen Wert (13) einer anderen Kennung, der die Identifikation eines optometrischen Geräts (15) an einem ersten optometrischen Messort (10) gestattet, beinhaltet;
(b) Vornehmen, mit Hilfe des optometrischen Geräts (15) des ersten Orts (10), mindestens einer optometrischen Messung (16), die mindestens eine Messung eines Augenrefraktionsparameters des Brillenträgers (1) und/oder eine Messung der vertikalen und horizontalen Position der Pupille des Auges dieses Trägers in Bezug auf ein Gestell, das dazu bestimmt ist, die Korrekturlinse aufzunehmen, beinhaltet;
(c) Übertragen, an einen zweiten Ort (20), eines Satzes digitaler Messdaten (18), der das optometrische Messergebnis (16) des Schritts (b) beinhaltet, wobei der Satz digitaler Messdaten (18) mit der ersten Registrierung (14) datentechnisch verbunden ist;
(d) digitales Verarbeiten des optometrischen Messergebnisses (16), das mit der ersten Registrierung (14) datentechnisch verbunden ist, in Abhängigkeit von einem Repositorium digitaler Daten (45) und jeweiligen Kennungen des Brillenträgers (12) und des optometrischen Geräts (13) der ersten Registrierung (14), wobei das Repositorium digitaler Daten ein Repositorium optometrischer Messungen des Brillenträgers (12) und des optometrischen Geräts (15) beinhaltet und wobei die digitale Verarbeitung das Vergleichen und Bestimmen einer Abweichung zwischen der optometrischen Messung (16) und dem Repositorium optometrischer Messungen beinhaltet, um der optometrischen Messung (16) je nachdem, ob diese Abweichung kleiner oder größer als eine vorbestimmte Schwelle ist, entweder ein Validierungskriterium oder ein Nichtvalidierungskriterium zuzuordnen;
(g) Übertragen, an den ersten Ort (10), des Nichtvalidierungskriteriums, um die optometrische Messung (16) zu wiederholen, bzw. des Validierungskriteriums, um die optometrische Messung (16) zu akzeptieren;
(i) Erzeugen, an dem dritten Ort (30), eines Satzes digitaler Auftragsdaten für eine Korrekturlinse, wodurch die Fertigung dieses Glases ausgelöst wird.

2. Verfahren nach Anspruch 1, wobei Schritt (d) einen Schritt des Kalibrierens des optometrischen Messergebnisses (16) beinhaltet.

3. Verfahren nach einem der Ansprüche 1 bis 2, wobei Schritt (d) einen Schritt des Registrierens des Bewertungskriteriums in einer Datenbank (41) umfasst.

4. Verfahren nach einem der Ansprüche 1 bis 3, wobei die erste Registrierung (14) nach Abschluss von Schritt (a) in einer Datenbank (41) registriert wird, der Satz digitaler Messdaten (18) nach Abschluss von Schritt (b) in derselben Datenbank (41) registriert wird und das Ergebnis der digitalen Verarbeitung nach Abschluss von Schritt (d) in derselben Datenbank (41) registriert wird.

5. Verfahren nach einem der Ansprüche 3 oder 4, wobei Schritt (c) des Übertragens des Satzes digitaler Messdaten (18) einen Schritt des Registrierens einer digitalen Signatur in der ersten Registrierung (14) der Datenbank umfasst.

6. Verfahren nach einem der Ansprüche 1 bis 5, ferner beinhaltend mindestens eine weitere Ausführung des Verfahrens nach Anspruch 1, wobei diese weitere Ausführung mit demselben Wert einer ersten Kennung, der demselben Brillenträger entspricht, verbunden ist.

7. Verfahren nach einem der Ansprüche 1 bis 6, ferner beinhaltend die folgenden Schritte:
(e) Bestimmen eines Satzes digitaler Daten einer Sehkorrekturverschreibung einer neuen Korrekturlinse in Abhängigkeit von dem Satz digitaler Daten der signierten Messungen,
(f) Validieren und digitales Signieren des Satzes digitaler Daten einer Sehkorrekturverschreibung durch eine Fachkraft, die befähigt ist, die Refraktionsstärken einer neuen Korrekturlinse zu verschreiben,
(g) Übertragen, an einen dritten Ort (30), des Satzes digitaler Verschreibungsdaten, der mit der ersten Kennung (12) des Trägers und mit einer mit der befähigten Fachkraft verknüpften Signatur assoziiert ist,
(h) Zertifizieren der in Schritt g) übertragenen Signatur und Übertragen des Ergebnisses dieser Zertifizierung an den dritten Ort.

8. Verfahren nach einem der Ansprüche 1 bis 7, ferner beinhaltend den folgenden Schritt:
(j) Auswählen des optischen Designs oder der Kategorie des optischen Designs oder Anpassen der Berechnung des Designs in Abhängigkeit von der mit der befähigten Fachkraft verknüpften Signatur.

9. Verfahren nach Anspruch 8, ferner beinhaltend die folgenden Schritte:
(k) datentechnisches Registrieren einer Bilddatei einer bereits bestehenden Sehkorrekturverschreibung des Trägers, die mit der ersten Kennung des Trägers assoziiert ist;
(1) Übertragen, an den zweiten Ort, eines Satzes digitaler Messdaten, der das Ergebnis der Messung des Schritts (b) beinhaltet, und der Bilddatei einer bereits bestehenden Verschreibung, die mit der ersten Kennung des Trägers und mit einer mit dem ersten Ort oder mit dem optometrischen Gerät verknüpften Signatur assoziiert sind;
(m) Bestimmen, durch Verarbeitung der Bilddatei einer bereits bestehenden Verschreibung, des Satzes digitaler Sehkorrekturverschreibungsdaten, der die drei Refraktionsstärken der neuen Korrekturlinse beinhaltet.

## Claims

1. Method for controlling the quality of optometric measurements for determining the optical-mechanical properties and the quality of a corrective spectacle lens suitable for a spectacle wearer, the controlling method comprising the following steps:
(a) computationally recording a first recording (14) comprising at least one first value (12) of a first identifier allowing a spectacle wearer (1) to be identified and at least one other value (13) of another identifier allowing an optometric apparatus (15) at a first optometric measurement site (10) to be identified;
(b) carrying out, by means of the optometric apparatus (15) of the first site (10), at least one optometric measurement (16) comprising at least one measurement of an ocular refraction parameter of the spectacle wearer (1) and/or a measurement of horizontal and vertical positions of the pupil of the eye of this wearer relative to a frame intended to accommodate the corrective lens;
(c) transmitting to a second site (20) a numerical measurement dataset (18) comprising the optometric measurement (16) result of step (b), the numerical measurement dataset (18) being computationally associated with the first recording (14);
(d) digitally processing the optometric measurement (16) result computationally associated with the first recording (14) depending on numerical reference data (45) and respective identifiers of the spectacle wearer (12) and of the optometric apparatus (13) of the first recording (14), the numerical reference data comprising an optometric reference measurement of the spectacle wearer (12) and of the optometric apparatus (15), and the digital processing comprising comparison and determination of a deviation between said optometric measurement (16) and the optometric reference measurement, to assign either a validation criterion or a non-validation criterion to said optometric measurement (16) depending on whether this deviation is less than or greater than a predetermined threshold;
(g) transmitting to the first site (10) said non-validation criterion to repeat said optometric measurement (16) or, respectively, the validation criterion to accept said optometric measurement (16); and (i) generating at the third site (30) a digital corrective-lens order dataset triggering the manufacture of this eyeglass.

2. Method according to Claim 1, wherein step (d) comprises a step of calibrating said optometric measurement (16) result.

3. Method according to one of Claims 1 to 2, wherein step (d) includes a step of recording the evaluation criterion in a database (41).

4. Method according to one of Claims 1 to 3, wherein the first recording (14) is recorded in a database (41) at the end of step (a), the numerical measurement dataset (18) is recorded in the same database (41) at the end of step (b) and the result of the digital processing is recorded in the same database (41) at the end of step (d) .

5. Method according to either of Claims 3 and 4, wherein step (c) of transmitting the numerical measurement dataset (18) includes a step of recording a digital signature in the first recording (14) of the database.

6. Method according to one of Claims 1 to 5, furthermore comprising at least one other execution of the method of Claim 1, this other execution being associated with the same first identifier value corresponding to the same spectacle wearer.

7. Method according to one of Claims 1 to 6, furthermore comprising the following steps:
(e) determining a numerical visual-correction prescription dataset of a new corrective lens depending on the numerical dataset of the signed measurements;
(f) having the numerical visual-correction prescription dataset digitally signed and validated by a professional qualified to prescribe the refraction powers of a new corrective lens;
(g) transmitting to a third site (30) the numerical prescription dataset associated with the first identifier (12) of the wearer and with a signature attached to the qualified professional; and
(h) certifying the signature transmitted in step g) and transmitting the result of this certification to the third site.

8. Method according to one of Claims 1 to 7, furthermore comprising the following step:
(j) selecting the optical design or the category of the optical design or adapting the calculation of the design depending on the signature attached to the qualified professional.

9. Method according to Claim 8, furthermore comprising the following steps:
(k) computationally recording an image file of a pre-existing visual-correction prescription of the wearer associated with the first identifier of the wearer;
(1) transmitting to the second site a numerical measurement dataset comprising the result of the measurement of step (b) and the image file of a pre-existing prescription, which are associated with the first identifier of the wearer and with a signature attached to the first site or to the optometric apparatus; and
(m) determining by processing of the image file of a pre-existing prescription the numerical visual-correction prescription dataset comprising the three refraction powers of the new corrective lens.
